# EUROPEAN PATENT APPLICATION

(11) **EP 1 106 189 A2**
(43) Date of publication of application: **13.06.2001**
(21) Application number: 00127080.0
(22) Date of filing: 11.12.2000
(51) Int. Cl.: A61L 24/10, A61L 24/08, A61L 24/04

(54) **Photo-curing tissue adhesive**

(30) Priority: 09.12.1999 JP 35026999
(71) Applicant: Kabushiki Kaisha Vayu, Nagoya-shi, Aichi-ken (JP); Matsuda, Takehisa, Fukuoka-shi, Fukuoka-ken (JP); Nakayama, Yasuhide, Toyonaka-shi, Osaka-fu (JP)
(72) Inventor: Matsuda, Takehisa, Fukuoka-shi, Fukuoka-ken (JP); Nakayama, Yasuhide, Toyonaka-shi, Osaka-fu (JP); Tsutsui, Nobumasa, Nagoya-shi, Aichi-ken (JP)
(74) Representative: Leson, Thomas Johannes Alois, Dipl.-Ing.

(57) **Abstract**

In order to provide an adhesive to adhere to the tissue of organisms, a buffered solution containing a protein macromer having a vinylated protein, a polysaccharide macromer having a vinylated polysaccharide, or the both by mixture, along with a photo-reactive compound which generates radicals by irradiation is prepared. The solution is cured into a gel state by irradiation, adheres to and is fixed on the tissue surface.

## Description

### FIELD OF THE INVENTION

This invention relates to a tissue adhesive for medical treatment, more particularly to a photo-curing tissue adhesive for being adhered to and fixed on the organic tissue by curing a water solution which contains a protein or polysaccharide macromer having a vinyl group into a gel state with use of a photo-reactive compound which generates radicals by irradiation.

### BACKGROUND OF THE INVENTION

In joining of soft tissues such as cutes and organs in surgery, it usually takes 1-2 weeks to replace the damaged tissue and heal the wound by self-anagenesis healing mechanism. During that period, it is necessary to maintain the joining force to bear the beat pressure, the contractive force, or the external force.

Conventionally, anastomosis by surgical needle and surgical suture has been generally performed in joining of tissues. As an alternative tissue-joining method, there has been a method using adhesives and the following adhesives have been put into practical use: (1) a cyanoacrylate-based adhesive which utilizes the mechanism that liquid cyanoacrylate monomer polymerizes and cures in a short time with addition of moisture; (2) a fibrin glue which utilizes blood clotting mechanism of organisms that fibrinogen forms insoluble fibrin clot by the function of thrombin; and (3) a gelatin-based adhesive which cross-links gelatin and resorcinol with formalin.

In the anastomosis by surgical needle and surgical suture, there is a problem not only in that it could be difficult to suture depending on the region of the lesion, but also of blood flow damage in the peripheral tissue by fastening of the surgical suture, necrosis, and bleeding from the surgical suture hole. While the cyanoacrylate-based adhesives are superior in quick curing and adhesion to tissue, the cured product lacks flexibility. In addition, because it is quite harder than the soft tissue, it is likely to cause joining deficiency by organic reaction such as shrinking of tissue, and disturbs wound healing. Moreover, because the degradation of adhesives in organisms takes about a half year to one year, it can be wrapped by normal tissue and become a foreign body. When degraded, it may generate highly toxic formaldehyde as well. The fibrin glue is inferior in adhesion to organic tissue. Because it cannot follow the motion of tissues, it is easy to peel off. In addition, since it is a human-derived blood product, it is afraid that infection such as hepatitis or acquired immunodeficiency syndrome may occur. Moreover, it is more expensive than other tissue adhesives and cannot be used in bulk. The gelatin-based adhesive shows high adhesion to tissue, but it is said that the formalin which cross-links gelatin with resorcinol becomes toxic because it also cross-links with a protein in organisms.

### SUMMARY OF THE INVENTION

An object of the present invention is to provide a tissue adhesive for medical treatment which is made of cheap raw materials, and which can be adhered tightly to organic tissue by quickly being cured on the moist tissue surface by convenient irradiation without use of toxic chemicals, of which curing product being flexible and having biodegradability, and of which degraded product being nontoxic.

In order to attain the above object, a photo-curing tissue adhesive of the present invention is a water solution containing a protein macromer having a vinylated protein, a polysaccharide macromer having a vinylated polysaccharide, or the both by mixture, along with a photo-reactive compound which generates radicals by irradiation, or the solution to which a vinyl compound is further added, and adheres to tissue by being photo-cured into a gel state.

Specific features of the photo-curing tissue adhesive of the present invention are: (1) it can be quickly cured by irradiation of light, (2) it has adhesion to tissue under the presence of liquid such as body fluid or blood, (3) cured gel has physical flexibility to follow the motion of organic tissue and the flexibility can be easily adjusted by changing the kind and the amount of the protein macromer or polysaccharide macromer and the vinyl compound to be used, (4) it is superior in biocompatibility because of use of nontoxic macromolecule derived from organisms, and (5) it has biodegradability and the degraded product is nontoxic.

The photo-curing tissue adhesive of the present invention is a solution which contains a protein or polysaccharide macromer, and a vinyl compound by the weight ratio of 100:0-1:99, preferably by the ratio of 2:1-1:2, the macromer and the vinyl compound mixed with water, physiological saline water solution, or balanced saline water solution by the concentration ratio of 1-99.9%, preferably by the ratio of 30%. As for the photo-reactive compound, 0.0001-30%, preferably 0.5% of the total weight of the protein macromer or polysaccharide macromer, and the vinyl compound contained in the solution is mixed.

A protein or a protein of the protein macromer of the present invention is organism-derived collagen, albumin, fibronectin, and gelatin which is the degenerated body, or artificially synthesized complex polypeptide, preferably gelatin. A polysaccharide or a polysaccharide of the polysaccharide macromer is organism-derived heparin, glycosaminoglycan, cellulose and starch, or their generated bodies, and artificially synthesized complex polypeptide, preferably heparin. A vinyl group of the vinylated protein can be any group having double bond in the molecule such as an acrylate group and a styrene group, preferably a styrene group. It is preferred that the vinylation of the protein is chemical bonding by amide bonding using water-soluble carbodiimide bonding reagent such as 1-ethyl-3-(-dimethylaminopropyl) carbodiimide hydrochloride and 1 cyclohexyl-3- (2-morpholinoethyl) carbodhmidometho-p-toluenesulfonic acid. However, it can be chemical bonding by bivalent cross-linking reagent such as ethyl chloroformate, carbonyldiimidazole, dimethyladipinimitate, and disuccinimidyl suberate. As for the bonding between the protein and the vinyl group, polyethylene glycol chain or long-chain alkyl chain can be applied as a spacer.

The vinyl compound used in the present invention can be any radically-polymerized, water-soluble vinyl monomer, oligomer and polymer, and it is also preferred that it is a polyfunctional vinyl compound such as a bifunctional and trifunctional compound. It is most preferable if it is polyethyleneglycol diacrylate.

The photo-reactive compound is an organic compound which generates radicals by irradiation of light. For example, it is at least one that is selected from a group consisting of: a carbonyl compound such as camphorquinone, acetophenone, benzophenone, dimethoxyphenyl acetophenone, and the derivatives; a sulfur compound such as dithiocarbamate, xanthate, tbiophenol, and the derivatives; peroxide such as benzoyl peroxide, butyl peroxide, and the derivatives; an azobis compound such as azobisisobutyronitrile, azobis isobutyric acid ester, and the derivatives; a halogenated compound such as bromopropane, chloromethyl naphthalene, and the derivatives; an azide compound such as phenyl azide, and the derivatives; xanthene dye such as rhodamine, erythron, fluorescein, eosin, and the derivatives; and riboflavin and the derivatives, or the one to which proton donor such as amines and alcohols is further added. Preferably, it is sole camphorquinone or the same to which dimethylaminoethyl methacrylate is further added. In case that the photo-reactive compound is a photo-reactive protein or polysaccharide in which tile above-mentioned photo-reactive compound is chemically introduced in the side chain of the protein or polysaccharide, even the water solution containing the photo-reactive protein or polysaccharide without containing the previously-mentioned protein macromer or polysaccharide macromer can be used as adhesive.

The mixed water solution is physiological saline water solution such as Ringer's solution and Rock's solution, and balanced saline water solution such as phosphoric acid buffer solution, Tyrode' s Solution, Hanks' solution, Earle' s solution, HEPES soluction, preferably physiological saline.

The light source for irradiation is a halogen lamp, a xenon lamp, an incandescent lamp, a mercury lamp, excimer laser, argon ion laser, preferably a halogen lamp having the wavelength ranging from 300 to 500nm. The time period for the irradiation is preferably about 1 minute.

Curing of the photo-curing tissue adhesive of the present invention proceeds as follows: firstly, radicals are generated when the photo-reactive compound is irradiated; then, polymerization of the vinyl group contained in the protein or polysaccharide macromer occurs by the generated radicals; in case that the vinyl compound coexists, copolymerization with the protein macromer and polysaccharide macromer also occurs; and finally, by the polymerization(s), crosslinking occurs between the proteins or the polysaccharides, and between the vinyl compounds, and a cured product in gel state is generated.

The present invention provides a tissue adhesive for medical treatment comprising a solution containing a protein macromer having at least a vinyl group in one part of the protein, polysaccharide macromer having at least a vinyl group in one part of the polysaccharide, or the both by mixture, along with a photo-reactive compound, cured by irradiation of light into a gel state and adhered to the organic tissue. The tissue adhesive of the present invention, without giving any serious lesions to the organic tissue, adheres quickly to or joins the organic tissue just by irradiation. In addition, components of the tissue adhesive, the cured product and the degraded product are all nontoxic. The tissue adhesive of the present invention can offer not only the simplification and shortening of the operation but also new techniques which could not be performed under the suturing method, such as hemostasis of the parenchymatous organ such as the exfoliated region of adhesion, liver and spleen, or small blood vessels, or the filling to the dead space.

### BRIEF DESCRIPTION OF THE DRAWING FIGURES

The invention will now be described, by way of example, with reference to the accompanying drawings, in which:

Fig. 1 is an explanatory view showing a synthetic pathway of styrenated gelatin which is a kind of protein macromer.

Fig. 2A and Fig. 2B are graphs indicating the curing state of a photo-curing tissue adhesive under irradiation.

Fig. 3A and Fig. 3B are graphs indicating the enzymatic degradation of a photo-cured product.

Fig. 4A and Fig. 4B are explanatory views showing the joining of a dog's thoratic aorca using the photo-curing tissue adhesive.

Fig. 5 is an explanatory view showing a cross section of the joined tissue of the dog' sthoratic aorca.

Fig. 6 is an explanatory view showing hemostasis of a rat' sliver.

Fig. 7 is an explanatory view showing a cross section of the rat' s liver from which bleeding is stopped.

Fig. 8 shows the degradation state inside an organism of the product photo-cured on the liver surface.

### DETAILED DESCRIPTION OF THE PREFERRED EMBODIMENT

### Example 1: Synthesis of gelatin macromer

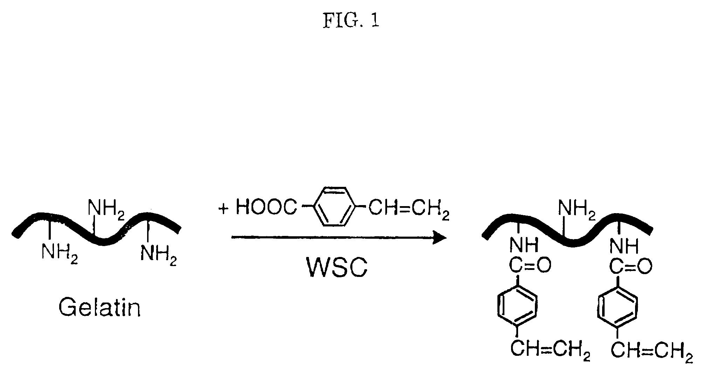

Fig. 1 shows a synthetic pathway of styrenated gelatin having a styrene group in the side chain as gelatin macromer. 50ml of a solution containing 0.57g (3.4mmol) of 4-vinyl benzoic acid was cooled to 0°C, and 1,48g (7.7mmol) of 1-ethyl-3-(3-dimethylaminopropyl) carbodiimide hydrochloride was added to it. After stirring the solution at 0°C for an hour, 50ml of a phosphoric acid buffer solution in which 1g (0.01mmol) of gelatin is dissolved was also added, and the stirring was continued at room temperature for a night. After dialyzing the solution for three days using a cellulose tube under running water, freeze drying was performed, and 0.99g of white cotton-like gelatin macromer was obtained. The amount of the styrene group introduced was calculated by spectrometry and was about 24 groups per a gelatin molecule.

### Example 2: Preparation of photo-curing tissue adhesive

By mixing 200mg of styrenated gelatin produced by Example 1 and 100mg of polyethylene glycol diacrylate having approximately 1000 molecular weight with 700 µl of physiological saline, a photo-curing tissue adhesive was prepared.

### Embodiment 1: Photo-curability of photo-curing tissue adhesive

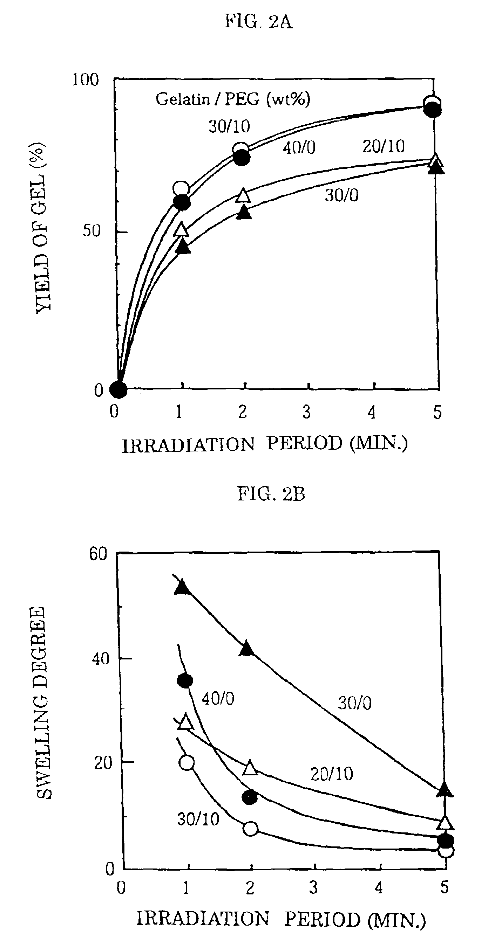

100mg of the photo-curing adhesive prepared by Example 2 was irradiated by a halogen lamp. Fig. 2A and 2B show the yield of gel and the swelling degree of the generated gel according to the irradiation time. As the irradiation time was increased, the yield of gel was increased and the swelling degree was decreased. It is apparent that the longer the irradiation time is, the more the gel of high strength is generated.

### Experiment 1: Degradability of photo-cured tissue adhesive

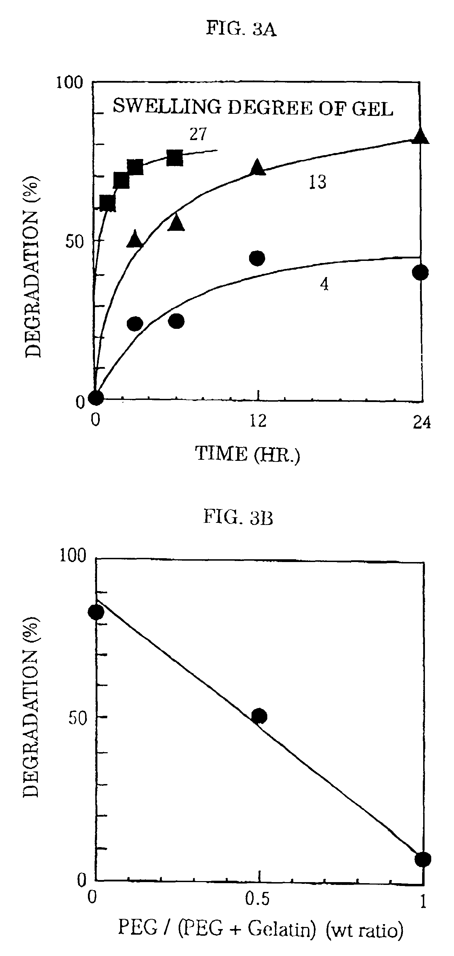

The degradability of the photo-cured tissue adhesive was evaluated by the enzymatic degradation using collagenase. After adding 20mg of the dry cured product to 2ml of a phosphoric acid buffer solution to swell, 1000unit of collagenase was added to it. After shaking it at 30°C for a predetermined period, undegraded solid residue was removed by filtering the reacted solution. Fig. 3A and 3B show the total organic carbon concentration, measured by TOC measuring apparatus, of the degraded product being dissolved in the filtered solution. As the shaking period was increased, the TOC concentration in the solution was increased. The higher gelatin content the product had, the faster the degradation proceeded.

### Embodiment 2: Joining of a dog' saorta

The thoracic aorta of an anesthetized dog of mixed breed (having the weight of about 14kg) was exposed. After clamped at about 5cm intervals, the thoracic aorta was cut open. The interrupted suture was performed at about 3-4mm intervals using 7-0 suture thread made of polypropylene. To the wound, 20 µl of the photo-curing tissue adhesive prepared by Example 2 was dropped. Then ultraviolet rays directed by a quartz fiber was applied to the wound for one minute. The application of the adhesive and the irradiation of ultraviolet rays were repeated several times. The clamp on the peripheral side was firstly removed, and after it is admitted that there was no spilling of blood, then the clamp on the central side was removed one minute later.

### Experiment 2: Verification of tissue adhesiveness

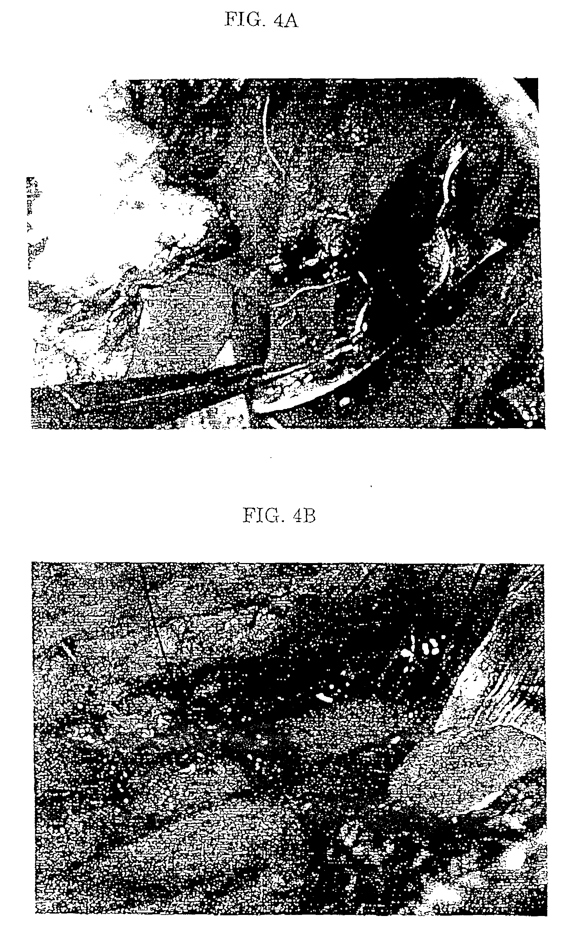

Fig. 4A and 4B show pictures of the blood tissue of the incised wound of the dogs s thoracic aorta after the joining. It is apparent that the product is quickly cured after the one-minute irradiation, and covers the wound. Because the beating of the aorta was started without showing any kind of bleeding after the blood flow was started, it is apparent that the cured body has enough flexibility to correspond to the motion of organism. The cured tissue adhesive was not exfoliated from the wound and remained after the wound was cleaned with physiological saline.

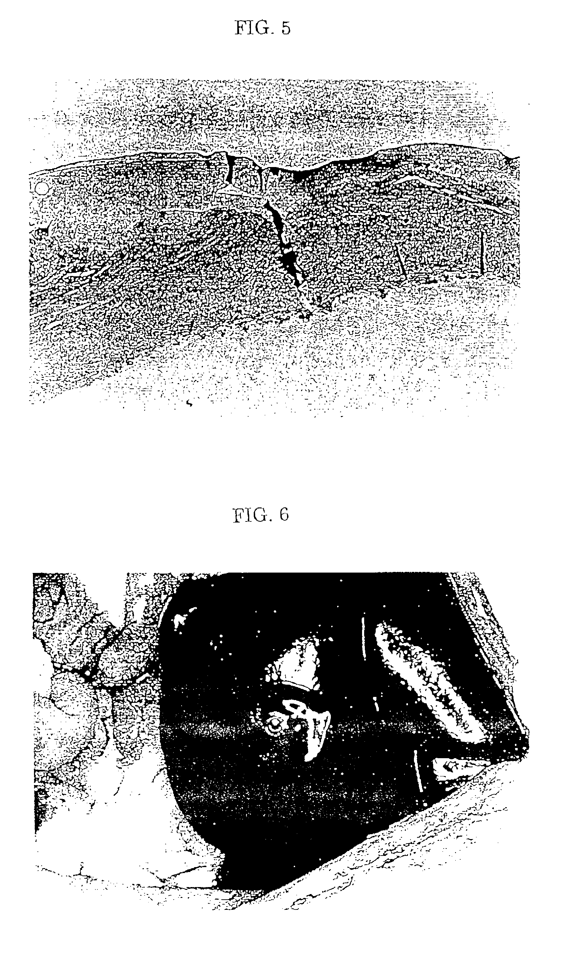

Fig. 5 shows a picture of the tissue taken by an optical microscope right after the joining. From the picture, it is apparent that the cured tissue adhesive was adhered to and cover the surface of the blood vessel tissue.

### Embodiment 3: Hemostasis of the surface of a rat' sliver

The abdomen of a male Wister rat (having the average weight of 250g) anesthetized by Nembutal was cut open and 200unit/kg heparin was injected from the vein of the rat' stail. The liver was exposed and bled by making an incised wound having a diameter of about 2mm and the deepness of about 2mm by a trepan. 10 *µ*l of the photo-curing tissue adhesive prepared by Example 2 was dropped on the wound. By radiating ultraviolet rays directed by a quartz fiber for one minute, the adhesive was cured on the wound and adhered to the liver tissue.

### Experiment 3: Verification of the tissue adhesiveness of the embodiment 3

Fig. 6 is a picture of the rat' sliver after the bleeding from the incised wound was stopped. Since bleeding cannot be seen from the wound, it is apparent that adhesion on the tissue surface where blood is present is possible. The cured tissue adhesive was not exfoliated from the wound after the wound was cleaned with physiological saline. The effect of the hemostasis was maintained.

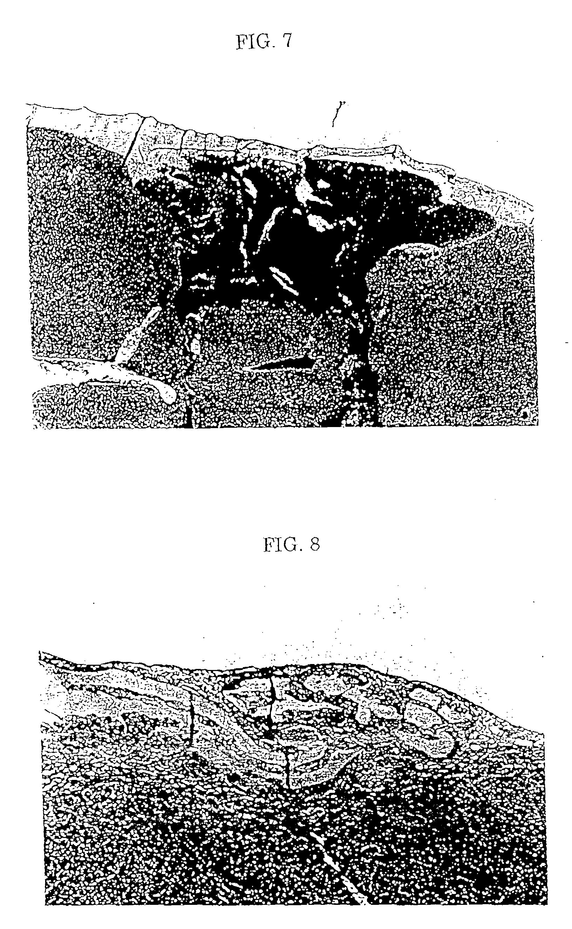

Fig. 7 is a picture of the liver tissue taken by an optical microscope right after the hemostasis. It is apparent that the cured tissue adhesive is adhered to and cover the liver surface and prevents bleeding.

Fig. 8 is a picture of the liver tissue taken by an optical microscope after one month from the hemostasis. It can be seen that the cured adhesive is fragmentized and miniaturized. It is apparent the cured product has biodegrability. Lesions such as degeneration and necrosis of the peripheral tissue were not seen.

In order to provide an adhesive to adhere to the tissue of organisms, a buffered solution containing a protein macromer having a vinylated protein, a polysaccharide macromer having a vinylated polysaccharide, or the both by mixture, along with a photo reactive compound which gendrates radicals by irradiation is prepared. The solution is cured into a gel state by irradiation, adheres to and is fixed on the tissue surface.

## Claims

1. A photo-curing tissue adhesive for medical treatment comprising a water solution containing a protein macromer having a vinyl group combined to at least one part of the protein and a photo-reactive compound by mixture, the solution being cured into a gel state by irradiation of light and adhering to the tissue of organisms.

2. A pboto-curing tissue adhesive for medical treatment comprising a water solution containing a polysaccharide macromer having a vinyl group combined to at least one part of the polysaccharide and a photo-reactive compound by mixture, the solution being cured into a gel state by irradiation of light and adhering to the tissue of organisms.

3. A photo-curing tissue adhesive for medical treatment set forth in claim 2, wherein said water solution further contains a protein macromer having a vinyl group combined to at least one part of the protein.

4. A photo-curing tissue adhesive set forth in one of claims 1-3, wherein said water solution further contains a vinyl compound.

5. A photo-curing tissue adhesive for medical treatment comprising a solution containing a compound in which a photo-reactive compound is chemically combined with a protein macromer, a polysaccharide macromer, or at least one of a protein and a polysaccharide, or containing at least two of the foregoings by mixture, the solution being cured into a gel state by irradiation of light and adhering to the tissue of organisms.

6. A photo-curing tissue adhesive set forth in claim 5, wherein said water solution further contains a vinyl compound.

7. A photo curing tissue adhesive set forth in one of claims 1-6, wherein said photo-reactive compound generates radicals by irradiation of light.

8. A photo-curing tissue adhesive set forth in one of claims 1-7, wherein said water solution comprises physiological saline water solution or balanced saline water solution.
